(19) Europäisches Patentamt
European Patent Office
Office européen des brevets

(11) Veröffentlichungsnummer: **0 052 579**
**B1**

(12) # EUROPÄISCHE PATENTSCHRIFT

(45) Veröffentlichungstag der Patentschrift: **14.11.84**

(51) Int. Cl.³: **C 07 D 211/46**, C 08 K 5/34 // C09D3/00

(21) Anmeldenummer: **81810449.9**

(22) Anmeldetag: **11.11.81**

(54) **Neue Lichtschutzmittel.**

(30) Priorität: **17.11.80 CH 8520/80**

(43) Veröffentlichungstag der Anmeldung: **26.05.82 Patentblatt 82/21**

(45) Bekanntmachung des Hinweises auf die Patenterteilung: **14.11.84 Patentblatt 84/46**

(84) Benannte Vertragsstaaten: **AT BE CH DE FR GB IT LI NL SE**

(56) Entgegenhaltungen:
**DE - A - 2 623 422**
**DE - A - 2 755 340**
**US - A - 4 021 432**

(73) Patentinhaber: **CIBA-GEIGY AG, Postfach, CH-4002 Basel (CH)**

(72) Erfinder: **Berner, Godwin, Dr., Waldhofstrasse 70, CH-4310 Rheinfelden (CH)**
Erfinder: **Rembold, Manfred, Dr., Maulbeerstrasse 7, CH-4058 Basel (CH)**
Erfinder: **Rody, Jean, Dr., Rütiring 82, CH-4125 Riehen (CH)**

**EP 0 052 579 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue Estergemische von Polyalkylpiperidinderivaten, ihre Herstellung, ihre Verwendung als Stabilisatoren für Kunststoffe sowie das damit stabilisierte Material.

Aus dem US-Patent 4 021 432 sind Polyalkylpiperidinester als Lichtschutzmittel für Kunststoffe bekannt, wie das Bis-(1,2,2,6,6-pentamethyl-4-piperidyl)-sebacat. Für die praktische Anwendung haben sich solche bekannten Stabilisatoren nicht in allen Fällen als zufriedenstellend erwiesen. Insbesondere bei Einsatz fester Substanzen, gegebenenfalls auch in Form von Lösungen, ergeben sich in der Praxis Nachteile, die z.B. bei der Herstellung lichtstabiler Lacke, wie Autolacke, zu Problemen führen. So sind die bekannten Lichtschutzmittel für die Anwendung in lösungsmittelarmen Lacksystemen (high solids) nicht geeignet.

Aufgabe der Erfindung war es, neue Lichtschutzmittel zu finden, die diese Nachteile nicht oder aber in wesentlich geringerem Masse zeigen, die insbesondere mit dem Substrat gut verträglich sind, gut in das Substrat eingearbeitet werden können und sich dabei schnell und homogen verteilen, wobei das Substrat wirksam und lang anhaltend gegen den schädlichen Einfluss von Licht geschützt wird.

Demgemäss betrifft die Erfindung Estergemische von Polyalkylpiperidinderivaten der Formeln I und II

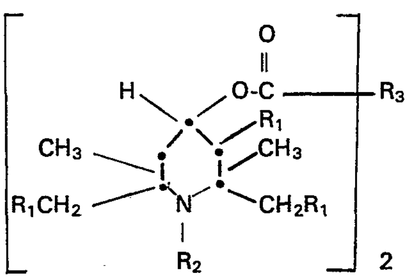

(I)

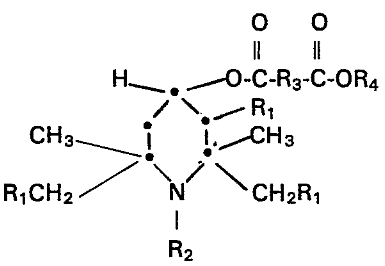

(II)

worin $R_1$ Wasserstoff oder Methyl ist, $R_2$ Wasserstoff, $C_{1-12}$ Alkyl, Allyl, $C_{7-11}$ Aralkyl, Cyanmethyl oder $C_{2-4}$ Acyl ist, $R_3$ $C_{1-18}$ Alkylen, $C_{2-18}$ Oxaalkylen, $C_{2-18}$ Thiaalkylen, $C_{2-18}$ Azaalkylen oder $C_{2-8}$ Alkylen ist und $R_4$ $C_{1-4}$ Alkyl ist.

$R_2$ ist als $C_{1-12}$ Alkyl insbesondere geradkettiges Alkyl mit insbesondere 1-4 C-Atomen, wie Äthyl, n-Propyl, n-Butyl, und vor allem Methyl.

$R_2$ ist als $C_{7-11}$ Aralkyl insbesondere Benzyl.

$R_2$ ist als $C_{2-4}$ Acyl insbesondere Alkanoyl oder Alkenoyl, wie Propionyl, Acryloyl und vor allem Acetyl.

$R_3$ ist als $C_{1-18}$ Alkylen verzweigtes oder insbesondere geradkettiges Alkylen, insbesondere solches mit 1-10 C-Atomen, wie Methylen, Äthylen, Trimethylen, Tetramethylen, Hexamethylen, Decamethylen und insbesondere Octamethylen.

$R_3$ ist als $C_{2-18}$ Oxaalkylen insbesondere geradkettiges Oxaalkylen mit insbesondere 2-9 C-Atomen, wie 2-Oxa-trimethylen oder 3-Oxa-pentamethylen.

$R_3$ ist als $C_{2-18}$ Thiaalkylen insbesondere geradkettiges Thiaalkylen mit insbesondere 2-9 C-Atomen, wie 2-Thia-trimethylen oder 3-Thia-pentamethylen.

$R_3$ ist als $C_{2-18}$ Azaalkylen geradkettiges oder verzweigtes, insbesondere am Aza verzweigtes Azaalkylen mit insbesondere 2-9 C-Atomen, wie 3-Aza-pentamethylen, 3-Aza-3-methyl-pentamethylen, 4-Aza-heptamethylen oder 4-Aza-4-methyl-heptamethylen.

$R_3$ ist als $C_{2-8}$ Alkylen insbesondere geradkettiges Alkenylen, wie Äthenylen oder 2-Buten-1,4-ylen.

$R_4$ ist als $C_{1-4}$ Alkyl z.B. Äthyl, n-Propyl, i-Propyl, n-Butyl, i-Butyl oder insbesondere Methyl.

Bevorzugt sind solche Estergemische der Formeln I oder II, worin
$R_1$ Wasserstoff ist,
$R_2$ $C_{1-4}$ Alkyl oder Benzyl ist,
$R_3$ $C_{1-18}$ Alkylen ist, und
$R_4$ $C_{1-4}$ Alkyl ist.

Besonders bevorzugt sind solche Estergemische der Formeln I und II, worin
$R_1$ Wasserstoff ist,
$R_2$ Methyl oder Benzyl ist,
$R_3$ geradkettiges $C_{2-10}$ Alkylen ist, und
$R_4$ $C_{1-4}$ Alkyl ist.

Insbesondere bevorzugt sind solche Estergemische der Formeln I und II, worin
$R_1$ Wasserstoff ist,
$R_2$ Methyl ist,
$R_3$ Octamethylen ist, und
$R_4$ Methyl ist.

Das Verhältnis der Ester I und II kann in weiten Grenzen variieren. Bevorzugt sind jedoch solche Estergemische, worin 98-60 Gew.-% I und 2-40 Gew.-% II vorhanden sind, insbesondere 90-70 Gew.-% I und 10-30 Gew.-% II.

Die erfindungsgemässen Estergemische können nach an sich bekannten Methoden hergestellt werden, so dadurch, dass man ein Piperidin der Formel III mit einem Diester der Formel IV

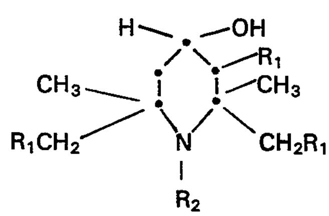

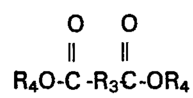

(III) (IV)

umsetzt, worin $R_1$, $R_2$, $R_3$ und $R_4$ die angegebenen Bedeutungen haben. Bei der Umsetzung wird vorzugsweise wie für Umesterungen üblich vorgegangen, wobei man bevorzugt auf 2 Mol III etwa 1,01 bis

1,5 Mol IV verwendet. Man kann aber auch, wenn erwünscht, die Komponenten I und III nach an sich bekannten Methoden getrennt herstellen, gegebenenfalls reinigen, und die Komponenten I und II dann mischen.

Die Ausgangsstoffe sind bekannt. Sollten einige von ihnen noch neu sein, so können sie in Analogie zu bekannten hergestellt werden.

Die Estergemische der Formeln I und II können gemäss der vorliegenden Erfindung als Stabilisatoren für Kunststoffe gegen deren Schädigung durch Einwirkung von Sauerstoff, Wärme und insbesondere Licht verwendet werden. Beispiele für solche Kunststoffe sind:

1. Polymere von Mono- und Diolefinen, beispielsweise Polyäthylen (das gegebenenfalls vernetzt sein kann), Polypropylen, Polyisobutylen, Polybuten-1, Polymethylpenten-1, Polyisopren oder Polybutadien sowie Polymerisate von Cycloolefinen wie z.B. von Cyclopenten oder Norbornen.

2. Mischungen der unter 1) genannten Polymeren, z.B. Mischungen von Polypropylen mit Polyäthylen oder mit Polyisobutylen.

3. Copolymere von Mono- und Diolefinen untereinander oder mit anderen Vinylmonomeren, wie z.B. Äthylen-Propylen-Copolymere, Propylen-Buten-1-Copolymere, Propylen-Isobutylen-Copolymere, Äthylen-Buten-1-Copolymere, Propylen-Butadien-Copolymere, Isobutylen-Isopren-Copolymere, Äthylen-Alkylacrylat-Copolymere, Äthylen-Alkylmethacrylat-Copolymere, Äthylen-Vinylacetat-Copolymere oder Äthylen-Acrylsäure-Copolymere und deren Salze (Ionomere), sowie Terpolymere von Äthylen mit Propylen und einem Dien, wie Hexadien, Dicyclopentadien oder Äthylidennorbornen.

4. Polystyrol.

5. Copolymere von Styrol oder α-Methylstyrol mit Dienen oder Acrylderivaten, wie z.B. Styrol-Butadien, Styrol-Acrylnitril, Styrol-Alkylmethacrylat, Styrol-Acrylnitril-Methylacrylat; Mischungen von hoher Schlagzähigkeit aus Styrol-Copolymeren und einem anderen Polymer wie z.B. einem Polyacrylat, einem Dien-Polymeren oder einem Äthylen-Propylen-Dien-Terpolymeren; sowie Block-Copolymere des Styrols, wie z.B. Styrol-Butadien-Styrol, Styrol-Isopren-Styrol, Styrol-Äthylen/Butylen-Styrol oder Styrol-Äthylen/Propylen-Styrol.

6. Pfropfcopolymere von Styrol, wie z.B. Styrol auf Polybutadien, Styrol und Acrylnitril auf Polybutadien, Styrol und Maleinsäureanhydrid und Polybutadien, Styrol und Alkylcrylate bzw. Alkylmethacrylate auf Polybutadien, Styrol und Acrylnitril auf Äthylen-Propylen-Dien-Terpolymeren, Styrol und Acrylnitril auf Polyalkylacrylaten oder Polyalkylmethacrylaten, Styrol und Acrylnitril auf Acryl-Butadien-Copolymeren, sowie deren Mischungen mit den unter 5) genannten Copolymeren, wie sie z.B. als sogenannte ABS, MBS, ASA oder AES-Polymere bekannt sind.

7. Halogenhaltige Polymere, wie z.B. Polychloropren, Chlorkautschuk, chloriertes oder chlorsulfoniertes Polyäthylen, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere wie Vinylchlorid-Vinylidenchlorid, Vinylchlorid-Vinylacetat oder Vinylidenchlorid-Vinylacetat.

8. Polymere, die sich von α,β-ungesättigten Säuren und deren Derivaten ableiten, wie Polyacrylate und Polymethacrylate, Polyacrylamide und Polyacrylnitrile.

9. Copolymere der unter 8) genannten Monomeren untereinander oder mit anderen ungesättigten Monomeren, wie z.B. Acrylnitril-Butadien-Copolymere, Acrylnitril-Alkylacrylat-Copolymere, Acrylnitril-Vinylchlorid-Copolymere, oder Acrylnitril-Alkylmethacrylat-Butadien-Terpolymere.

10. Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin.

11. Homo- und Copolymere von cyclischen Äthern, wie Polyalkylenglykole, Polyäthylenoxyd, Polypropylenoxyd oder deren Copolymere mit Bisglycidyläthern.

12. Polyacetale, wie Polyoxymethylen, sowie solche Polyoxymethylene, die Comonomere wie z.B. Äthylenoxyd enthalten.

13. Polyphenylenoxyde und -sulfide.

14. Polyurethane, die sich von Polyäthern, Polyestern und Polybutadien mit endständigen Hydroxylgruppen einerseits und aliphatischen oder aromatischen Polyisocyanaten andererseits ableiten sowie deren Vorprodukte.

15. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, Polyamid 6/10, Polyamid 11, Polyamid 12, Poly-2,4,4-trimethylhexamethylenterephthalamid, Poly-m-phenylen-isophthalamid, sowie deren Copolymere mit Polyäthern, wie z.B. Polyäthylenglykol, Polypropylenglykol oder Polytetramethylenglykol.

16. Polyharnstoffe, Polyimide und Polyamid-imide.

17. Polyester, die sich von Dicarbonsäuren und Dialkoholen und/oder von Hydroxycarbonsäuren oder den entsprechenden Lactonen ableiten, wie Polyäthylenterephthalat, Polybutylenterephthalat, Poly-1,4-dimethylolcyclohexanterephthalat, Polyhydroxybenzoate, sowie Block-Polyäther-ester, die sich von Polyäthern mit Hydroxylendgruppen ableiten.

18. Polycarbonate.

19. Polysulfone und Polyäthersulfone.

20. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.

21. Trocknende und nicht-trocknende Alkylharze.

22. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.

23. Vernetzbare Acrylharze, die sich von substituierten Acrylsäureestern ableiten, wie z.B. von

Epoxyacrylaten, Urethan-acrylaten oder Polyesteracrlyten.

24. Alkydharze, Polyesterharze und Acrylatharze, die mit Melaminharzen, Harnstoffharzen, Polyisocyanaten oder Epoxidharzen vernetzt sind.

25. Vernetzte Epoxidharze, die sich von Polyepoxiden ableiten, z.B. von Bis-glycidyläthern oder von cycloaliphatischen Diepoxiden.

26. Natürliche Polymere, wie Cellulose, Naturkautschuk, Gelatine, sowie deren polymerhomolog chemisch abgewandelte Derivate, wie Celluloseacetate, -propionate und butyrate, bzw. die Celluloseäther, wie Methylcellulose.

Von besonderer Bedeutung ist die Stabilisierung von Lacken.

Die Stabilisatoren werden den Kunststoffen in einer Konzentration von 0,01 bis 5 Gew.-%, berechnet auf das zu stabilisierende Material, zugesetzt. Vorzugsweise werden 0,03 bis 1,5, besonders bevorzugt 0,2 bis 0,6 Gew.-% der Verbindungen, berechnet auf das zu stabilisierende Material, in dieses eingearbeitet.

Die Einarbeitung kann nach der Polymerisation erfolgen, beispielsweise durch Einmischen der Verbindungen und gegebenenfalls weiterer Additive in die Schmelze nach den in der Technik üblichen Methoden, vor oder während der Formgebung, oder auch durch Aufbringen der gelösten oder dispergierten Verbindungen auf das Polymere, gegebenenfalls unter nachträglichem Verdunsten des Lösungsmittels.

Die neuen Gemische können auch in Form eines Masterbatches, der diese Verbindungen beispielsweise in einer Konzentration von 2,5 bis 25 Gew.-% enthält, den zu stabilisierenden Kunststoffen zugesetzt werden.

Im Falle von vernetztem Polyäthylen werden die Verbindungen vor der Vernetzung beigefügt.

Ausser den Gemischen der Formeln I und II können den Kunststoffen auch noch andere, bekannte Stabilisatoren bzw. Costabilisatoren zugesetzt werden. Dies können z.B. sein:

*1. Antioxidantien*

*1.1 Alkylierte Monophenole*

2,6-Di-tert.-butyl-4-methylphenol
2-Tert.-butyl-4,6-dimethylphenol
2,6-Di-tert.butyl-4-äthylphenol
2,6-Di-tert.butyl-4-n-butylphenol
2,6-Di-tert.butyl-4-i-butylphenol
2,6-Di-cyclopentyl-4-methylphenol
2-(α-Methylcyclohexyl)-4,6-dimethylphenol
2,6-Di-octadecyl-4-methylphenol
2,4,6-Tri-cyclohexylphenol

*1.2. Alkylierte Hydrochinone*

2,6-Di-tert.butyl-4-methoxyphenol
2,5-Di-tert.butyl-hydrochinon
2,5-Di-tert.amyl-hydrochinon

*1.3. Hydroxylierte Thiophenyläther*

2,2'-Thio-bis-(6-tert.butyl-4-methylphenol)
2,2'-Thio-bis-(4-octylphenol)
4,4'-Thio-bis-(6-tert.butyl-3-methylphenol)
4,4'-Thio-bis-(6-tert.butyl-2-methylphenol)

*1.4. Alkyliden-Bisphenole*

2,2'-Methylen-bis-(6-tert.butyl-4-methylphenol)
2,2'-Methylen-bis-(6-tert.butyl-4-äthylphenol)
2,2'-Methylen-bis-[4-methyl-6-(α-methylcyclohexyl)-phenol]
2,2'-Methylen-bis-(4-methyl-6-cyclohexylphenol)
2,2'-Methylen-bis-(6-nonyl-4-methylphenol)
2,2'-Methylen-bis-(4,6-di-tert.butylphenol)
2,2'-Äthyliden-bis-4,6-di-tert.butylphenol)
2,2'-Äthyliden-bis-(6-tert.butyl-4-isobutylphenol)
4,4'-Methylen-bis-(2,6-di-tert.butylphenol)
4,4'-Methylen-bis-(6-tert.butyl-2-methyl-phenol)
1,1-Bis-(5-tert.butyl-4-hydroxy-2-methylphenyl)-butan
2,6-Di-(3-tert.butyl-5-methyl-2-hydroxybenzyl)-4-methylphenol
1,1,3-Tris-(5-tert.butyl-4-hydroxy-2-methylphenyl)-butan
1,1-Bis-(5-tert.butyl-4-hydroxy-2-methylphenyl)-3-n-dodecyl-mercaptobutan
Äthylenglykol-bis-[3,3-bis-(3'-tert.butyl-4'-hydroxyphenyl)-butyrat]
Di-(3-tert.butyl-4-hydroxy-5-methylphenyl)-dicyclopentadien
Di-[2-(3'-tert.butyl-2'-hydroxy-5'-methyl-benzyl)-6-tert.butyl-4-methyl-phenyl]-terephthalat.

*1.5. Benzylverbindungen*

1,3,5-Tri-(3,5-di-tert.butyl-4-hydroxybenzyl)-2,4,6-trimethylbenzol
Di-(3,5-di-tert.butyl-4-hydroxybenzyl)-sulfid
3,5-di-tert.butyl-4-hydroxybenzyl-mercaptoessigsäure-isooctylester
Bis-(4-tert.butyl-3-hydroxy-2,6-dimethylbenzyl)dithiol-terephthalat
1,3,5-Tris-(3,5-di-tert.butyl-4-hydroxybenzyl)-isocyanurat
1,3,5-Tris-(4-tert.butyl-3-hydroxy-2,6-dimethylbenzyl)-isocyanurat
3,5-Di-tert.butyl-4-hydroxyphenyl-phosphonsäure-dioctadecylester
3,5-Di-tert.butyl-4-hydroxybenzyl-phosphonsäure-monoäthylester
Calcium-salz.

*1.6. Acylaminophenole*

4-Hydroxy-laurinsäureanilid
4-Hydroxy-stearinsäureanilid
2,4-Bis-octylmercapto-6-(3,5-di-tert.butyl-4-hydroxyanilino)-S-triazin

*1.7. Ester der β-(3,5-Di-tert.butyl-4-hydroxyphenyl)-propionsäure*

mit ein- oder mehrwertigen Alkoholen, wie z.B. mit

| | |
|---|---|
| Methanol | Diäthylenglykol |
| Octadecanol | Triäthylenglykol |
| 1,6-Hexandiol | Pentaerythrit |
| Neopentylglykol | Tris-hydroxyäthyl-isocyanurat |
| Thiodiäthylenglykol | Di-hydroxyäthyl-oxalsäurediamid |

*1.8. Ester der β-(5-tert.butyl-4-hydroxy-3-methylphenyl)-propionsäure*

mit ein- oder mehrwertigen Alkoholen, wie z.B. mit

| | |
|---|---|
| Methanol | Diäthylenglykol |
| Octadecanol | Triäthylenglykol |
| 1,6-Hexandiol | Pentaerythrit |
| Neopentylglykol | Tris-hydroxyäthyl-isocyanurat |
| Thiodiäthylenglykol | Di-hydroxyäthyl-oxalsäurediamid |

*1.9. Amide der β-(3,5-Di-tert.butyl-4-hydroxyphenyl)-propionsäure,* wie z.B.
N,N'-Di-(3,5-di-tert.butyl-4-hydroxyphenylpropionyl)-hexamethylendiamid
N,N'-Di-(3,5-di-tert.butyl-4-hydroxyphenylpropionyl)-trimethylendiamin
N,N'-Di-(3,5-di-tert.butyl-4-hydroxyphenylpropionyl-hydrazin.

*2. UV-Absorber und Lichtschutzmittel*

*2.1. 2-(2'-Hydroxyphenyl)-benztriazole,* wie z.B. das 5'-Methyl-, 3',5'-Di-tert.butyl-, 5'-Tert.butyl-, 5'-(1,1,3,3-Tetramethylbutyl)-, 5-Chlor-3',5'-di-tert.butyl-, 5-Chlor-3'-tert.butyl-5'-methyl-, 3'-sec.Butyl-5'-tert.butyl-, 4'-Octoxy-, 3',5-di-tert.-amyl-, 3',5'-Di-(1,1,3,3-tetramethylbutyl)-, 3',5'-Di-(α,α-dimethylbenzyl)-.
*2.2. 2-Hydroxybenzophenone,* wie z.B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.
*2.3. Ester von gegebenenfalls substituierten Benzoesäuren,* wie z.B. 4-tert.butyl-phenylsalicylat, Phenylsalicylat, Octylphenylsalicylat, Dibenzoylresorcin, Bis-(4-tert.butylbenzoyl)-resorcin, Benzoylresorcin, 3,5-Di-tert.butyl-4-hydroxybenzoesäure-2,4-di-tert.butyl-phenylester.
*2.4. Acrylate,* wie z.B. α-Cyan-β,β-diphenylacrylsäure-äthylester bzw. -isooctylester, α-Carbomethoxy-zimtsäuremethylester, α-Cyano-β-methyl-p-methoxy-zimtsäuremethylester bzw. -butylester, α-Carbomethoxy-p-methoxy-zimtsäure-methylester. N-(β-Carbomethoxy-β-cyanovinyl)-2-methyl-indolin.
*2.5. Nickelverbindungen,* wie z.B. Nickelkomplexe des 2,2'-Thio-bis-[4-(1,1,3,3-tetramethylbutyl)-phenols], wie der 1:1- oder der 1:2-Komplex, gegebenenfalls mit zusätzlichen Liganden wie n-Butylamin, Triäthanolamin oder N-Cyclohexyl-diäthanolamin, Nickeldibutyldithiocarbamat, Nickelsalze von 4-Hydroxy-3,5-di-tert.butylbenzyl-phosphonsäure-monoalkylester wie vom Methyl- oder Äthylester, Nickelkomplex von Ketoximen wie von 2-Hydroxy-4-methyl-phenyl-undecyl-ketonoxim, Nickelkomplexe des 1-Phenyl-4-lauroyl-5-hydroxy-pyrazols, gegebenenfalls mit zusätzlichen Liganden.
*2.6 Sterisch gehinderte Amine,* wie z.B. Bis-(2,2,6,6-tetramethylpiperidyl)-sebacat, Bis-(1,2,2,6,6-pentamethylpiperidyl)-sebacat, n-Butyl-3,5-tert.-butyl-4-hydroxybenzyl-malonsäure-bis-(1,2,2,6,6-pentamethylpiperidyl)-ester, Kondensationsprodukt aus 1-Hydroxyäthyl-2,2,6,6-Tetramethyl-4-hydroxypiperidin und Bernsteinsäure, Kondensationsprodukt aus N,N'-(2,2,6,6-Tetramethylpiperidyl)-hexamethylendiamin und 4-tert.ocytalamino-2,6-dichlor-1,3,5-s-triazin, Tris-(2,2,6,6-tetramethylpiperidyl)-nitrilotriacetat.
*2.7. Oxalsäurediamide,* wie z.B. 4,4'-Di-octyloxy-oxanilid, 2,2'-Di-octyloxy-5,5'-di-tert.butyl-oxanilid, 2,2'-Di-dodecyloxy-5,5'-di-tert.butyl-oxanilid, 2-Äthoxy-2'-äthyl-oxanilid, N,N'-Bis-(3-di-methylpropyl)-oxalamid, 2-Äthoxy-5-tert.butyl-2'-äthyl-oxanilid und dessen Gemisch mit 2-Äthoxy-2'-äthyl-5,4'-di-tert.butyl-oxanilid, Gemische von ortho- und para-Methoxy- sowie von o- und p-Äthoxy-di-substituierte Oxaniliden.

*3. Metalldesaktivatoren,* wie z.B. N,N'-Diphenyloxalsäurediamid, N-Salicylal-N'-salicyloylhydrazin, N,N'-Bis-salicyloylhydrazin, N,N'-Bis-(3,5-di-tert.-butyl-4-hydroxyphenylpropionyl)-hydrazin, 3-Salicyloylamino-1,2,4-triazol, Bis-benzyliden-oxalsäuredihydrazid.

*4. Phosphite und Phosphonite,* wie z.B. Triphenylphosphit, Diphenylalkylphosphite, Phenyldialkylphosphite, Tri-(nonylphenyl)-phosphit, Trilaurylphosphit, Trioctadecylphosphit, Distearyl-pentaerythritdiphosphit, Tris-(2,4-di-tert.butylphenyl)-phosphit, Diisodecyl-pentaerythrit-diphosphit, Di-(2,4-di-tert.butylphenyl)-pentaerythrit-diphosphit, Tristearyl-sobit-triphosphit, Tetrakis-(2,4-di-tert.-butylphenyl)-4m4'-biphenylen-diphosphonit.

*5. Peroxidzerstörende Verbindungen,* wie z.B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecylsulfid, Pentaerythrit-tetrakis-(β-dodecylmercapto)-propionat.

*6. Polyamidstabilisatoren,* wie z.B. Kupfersalze in Kombination mit Jodiden und/oder Phosphorverbindungen und Salze des zweiwertigen Mangans.

*7. Basische Co-Stabilisatoren,* wie z.B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Stearat, Na-Ricinoleat, K-Palmitat, Antimonbrenzcatechinat oder Zinnbrenzcatechinat.

*8. Nukleierungsmittel,* wie z.B. 4-tert.-Butylbenzoesäure, Adipinsäure, Diphenylessigsäure.

*9. Füllstoffe und Verstärkungsmittel,* wie z.B. Calciumcarbonate, Silikate, Glasfasern, Asbest, Talk, Kaolin, Glimmer, Bariumsulfat, Metalloxide und -hydroxide, Russ, Graphit.

*10. Sonstige Zusätze,* wie z.B. Weichmacher, Gleitmittel, Emulgatoren, Pigmente, optische Aufheller, Flammschutzmittel, Antistatika, Treibmittel.

Solche bekannte und übliche Zusatzstoffe sind nicht nur mit den Gemischen der Formeln I und II verträglich, sie können in einzelnen Fällen auch zu einer synergistischen Wirkungssteigerung führen.

Die Erfindung betrifft daher auch die durch Zusatz von 0,01 bis 5 Gew.-% eines Gemisches der Formeln I und II stabilisierten Kunststoffe, die gegebenenfalls noch andere bekannte und übliche Zusätze enthalten können. Die so stabilisierten Kunststoffe können in verschiedenster Form angewandt werden, z.B. als Folien, Fasern, Bändchen, Profile, Platten oder als spritzgegossene Artikel. Dabei können sie auch in verschäumter Form verwendet werden. Weiterhin können sie auch als Bindemittel für Lacke, Klebemittel oder Kitte sowie als Schicht- und Trägerstoffe in photographischen Materialien Verwendung finden.

Die folgenden Beispiele erläutern die Erfindung, ohne sie einzuschränken.

*Beispiel 1*

230,3 g (1 Mol) Sebacinsäuredimethylester und 308,3 g (1,8 Mol) 1,2,2,6,6-Pentamethyl-4-hydroxipiperidin werden mit 120 ml Xylol unter schwachem Stickstoffstrom auf ca. 135° erwärmt. Zur totalen Entwässerung des Gemisches werden ca. 20 ml Xylol abdestilliert. Man gibt nun zum Reaktionsgemisch 1 g Tetrabutylorthotitanat und erwärmt weiter unter schwachem Stickstoffstrom auf 130-40°. Es setzt sehr bald eine regelmässige Destillation von Methanol/Xylol ein. Die Innentemperatur wird innerhalb von 3-4 Stunden auf 160° gesteigert und der Kolbeninhalt bei dieser Temperatur weiter gerührt bis nach ca. 10 Stunden praktisch nichts mehr überdestilliert. Die letzten Spuren Lösungsmittel werden durch Destillation am Hochvakuum bei ca. 110° und 0,133 Pa entfernt und der Kolbenrückstand durch eine Glasfritte geklärt. Die erhaltene, schwach gelbliche, ölige Flüssigkeit ist ein Gemisch aus ca. 85% Sebacinsäure-bis-(1,2,2,6,6-pentamethyl-4-piperidinyl)-ester und ca. 15% Sebacinsäure-mono-(1,2,-2,6,6-pentamethyl-4-piperidinyl)-mono-methylester.

*Beispiel 2*

*Kristallisationsneigung der erfindungsgemässen Estergemische*

Die Kristallisationsneigung der erfindungsgemässen Estergemische ist abhängig vom Mischungsverhältnis der Komponenten I und II. Je nach Anwendung genügen geringe Anteile an Komponente II; wird jedoch ein stabiles flüssiges Estergemisch gewünscht, kann der Anteil an Komponente II leicht erhöht werden.

Als Komponente I wurde Sebacinsäure-bis-(1,2,-2,6,6-pentamethyl-4-piperidinyl)-ester und als Komponente II Sebacinsäure-mono-(1,2,2,6,6-pentamethyl-4-piperidinyl)-monomethyl-ester verwendet.

Die Estergemische werden bei 0°C und —20°C gelagert und die Zeit bis zum Durchkristallisieren gemessen. Längere Zeiten bedeuten erhöhte Stabilität der flüssigen Konsistenz der Estergemische.

| Anteil an Komponente II | Tage bis zum Durchkristallisieren bei 0°C | —20°C |
|---|---|---|
| 0% | 0 | 0 |
| 6% | 2-5 | 1-2 |
| 10% | 27-35 | |
| 17% | >90 | 15-20 |
| 20% | >90 | 25-30 |
| 24% | >90 | 60-70 |
| 65% | | >150 |

Überraschenderweise erfordert es einen ausserordentlich hohen und unerwünschten Lösungsmittelzusatz, um zu ähnlich befriedigenden Resultaten zu kommen .

**Patentansprüche**

1. Estergemische von Polyalkylpiperidinderivaten der Formeln I und II

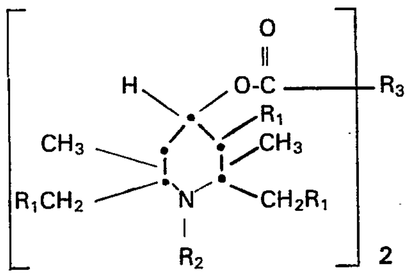

(I)

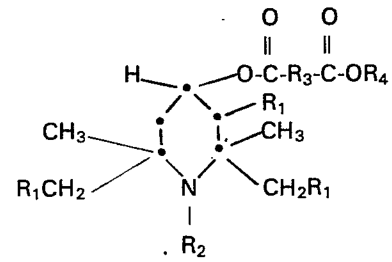

(II)

worin

$R_1$ Wasserstoff oder Methyl ist,

$R_2$ Wasserstoff, $C_{1-12}$ Alkyl, Allyl, $C_{7-11}$ Aralkyl, Cyanmethyl oder $C_{2-4}$ Acyl ist,

$R_3$ $C_{1-18}$Alkylen, $C_{2-18}$ Oxaalkylen, $C_{2-18}$ Thiaalkylen, $C_{2-18}$ Azaalkylen oder $C_{2-8}$ Alkylen ist, und

$R_4$ $C_{1-4}$ Alkyl ist

2. Estergemische nach Anspruch 1, worin

$R_1$ Wasserstoff ist,

$R_2$ $C_{1-4}$ Alkyl oder Benzyl ist,

$R_3$ $C_{1-18}$ Alkylen ist, und

$R_4$ $C_{1-4}$ Alkyl ist.

3. Estergemische nach Anspruch 1, worin

$R_1$ Wasserstoff ist,

$R_2$ Methyl oder Benzyl ist,

$R_3$ geradkettiges $C_{2-10}$ Alkylen ist, und

$R_4$ $C_{1-4}$ Alkyl ist.

4. Estergemische nach Anspruch 1, worin

$R_1$ Wasserstoff ist,

$R_2$ Methyl ist,

$R_3$ Octamethylen ist, und

$R_4$ Methyl ist.

5. Estergemische nach Anspruch 1, worin 98-60 Gew.-% I und 2-40 Gew.-% II vorhanden sind.

6. Estergemische nach Anspruch 1, worin 90-70 Gew.-% I und 10-30 Gew.-% II vorhanden sind.

7. Verwendung von Estergemischen der Formeln I und II gemäss Anspruch 1 zum Stabilisieren von Kunststoffen gegen photochemischen Abbau.

8. Verwendung gemäss Anspruch 7 zum Stabilisieren von Lacken.

9. Verfahren zum Stabilisieren von Kunststoffen gegen photochemischen Abbau durch Zusatz von 0,01 bis 5 Gew.-% eines Estergemisches der Formeln I und II gemäss Anspruch 1.

10. Gegen photochemischen Abbau stabilisierter Kunststoff, enthaltend als Stabilisator 0,1 bis 5 Gew.-% eines Estergemisches der Formeln I und II gemäss Anspruch 1.

11. Stabilisierter Kunststoff gemäss Anspruch 10, dadurch gekennzeichnet, dass es sich um einen Lack handelt.

12. Verfahren zur Herstellung von Estergemischen der Formeln I und II gemäss Anspruch 1, dadurch gekennzeichnet, dass man ein Piperidin der Formel III mit einem Diester der Formel IV

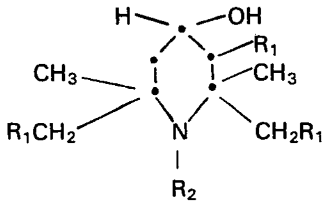

(III)

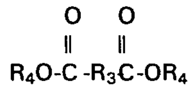

(IV)

umsetzt, worin $R_1$, $R_2$, $R_3$ und $R_4$ die angegebenen Bedeutungen haben.

**Claims**

1. An ester mixture of polyalkylpiperidine derivatives of the formulae I and II

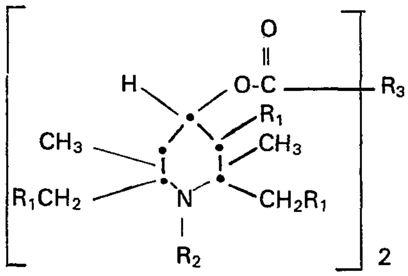

(I)

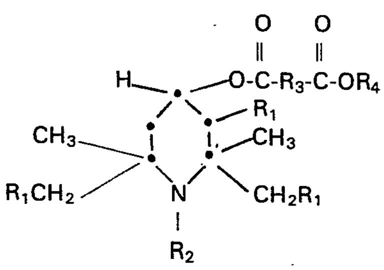

(II)

in which $R_1$ is hydrogen or methyl, $R_2$ is hydrogen, $C_{1-12}$ alkyl, allyl, $C_{7-11}$ aralkyl, cyanomethyl or $C_{2-4}$ acyl, $R_3$ is $C_{1-18}$ alkylene, $C_{2-18}$ oxaalkylene, $C_{2-18}$ thiaalkylene, $C_{2-18}$ azaalkylene or $C_{2-8}$ alkenylene, and $R_4$ is $C_{1-4}$ alkyl.

2. A mixture of esters according to claim 1, in which $R_1$ is hydrogen, $R_2$ is $C_{1-4}$ alkyl or benzyl, $R_3$ is $C_{1-18}$ alkylene and $R_4$ is $C_{1-4}$ alkyl.

3. A mixture of esters according to claim 1, in which $R_1$ is hydrogen, $R_2$ is methyl or benzyl, $R_3$ is straight-chain $C_{2-10}$ alkylene and $R_4$ is $C_{1-4}$ alkyl.

4. A mixture of esters according to claim 1, in which $R_1$ is hydrogen, $R_2$ is methyl, $R_3$ is octamethylene and $R_4$ is methyl.

5. A mixture of esters according to claim 1, in which 98-60% by weight of I and 2-40% by weight of II are present.

6. A mixture of esters according to claim 1, in which 90-70% by weight of I and 10-30% by weight of II are present.

7. The use of a mixture of esters of the formulae I and II according to claim 1 for stabilising plastics against photochemical degradation.

8. Use according to claim 7, for stabilising lacquers.

9. A process for stabilising plastics against photochemical degradation, by the addition of 0.01 to 5% by weight of a mixture of esters of the formulae I and II according to claim 1.

10. A plastic material which has been stabilised against photochemical degradation and which contains, as stabiliser, 0.1 to 5% by weight of a mixture of esters of the formulae I and II according do claim 1.

11. A stabilised plastic material according to claim 10, wherein said plastic material is a lacquer.

12. A process for the preparation of a mixture of esters of the formulae I and II according to claim 1, which comprises reacting a piperidine of the formula III with a diester of the formula IV

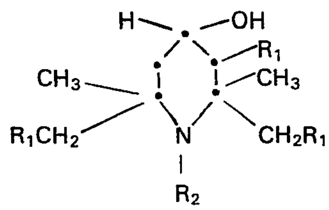

**(III)**

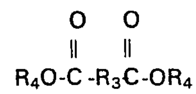

**(IV)**

in which $R_1$, $R_2$, $R_3$ and $R_4$ have the given meanings.

**Revendications**

1. Mélanges d'esters de dérivés de polyalkylpipéridines de formules I et II:

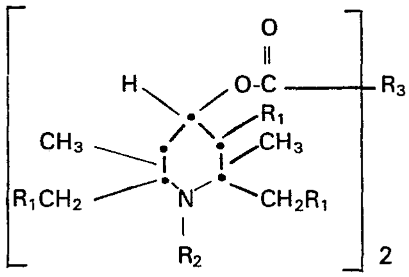

(I)

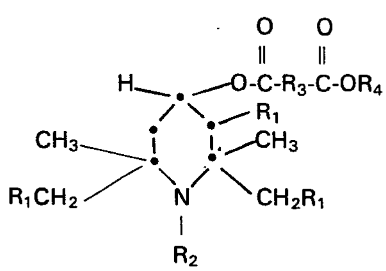

(II)

dans lesquelles:

$R_1$ désigne l'hydrogène ou un méthyle,

$R_2$ l'hydrogène, un alkyle en $C_{1-12}$, un allyle, un aralkyle en $C_{7-11}$, un cyanométhyle ou un acyle en $C_{2-4}$,

$R_3$ alkylène en $C_{1\text{-}18}$, un oxaalkylène en $C_{2\text{-}18}$, un thiaalkylène en $C_{2\text{-}18}$, un azaalkylène en $C_{2\text{-}18}$ ou un alcénylène en $C_{2\text{-}8}$, et

$R_4$ un alkyle en $C_{1\text{-}4}$.

2. Mélanges d'esters selon la revendication 1 dans lesquels:

$R_1$ est l'hydrogène,

$R_2$ un alkyle en $C_{1\text{-}4}$ ou un benzyle,

$R_3$ un alkylène en $C_{1\text{-}18}$, et

$R_4$ un alkyle en $C_{1\text{-}4}$.

3. Mélanges d'esters selon la revendication 1 dans lesquels:

$R_1$ est l'hydrogène,

$R_2$ un méthyle ou un benzyle,

$R_3$ un alkylène à chaîne droite en $C_{2\text{-}10}$, et

$R_4$ un alkyle en $C_{1\text{-}4}$.

4. Mélanges d'esters selon la revendication 1 dans lesquels:

$R_1$ est l'hydrogène,

$R_2$ un méthyle,

$R_3$ un octaméthylène, et

$R_4$ un méthyle.

5. Mélanges d'esters selon la revendication 1 contenant 98 à 60% en poids de la composante I et 2 à 40% de la composante II.

6. Mélanges d'esters selon la revendication 1 contenant 90 à 70% en poids de la composante I et 10 à 30% de la composante II.

7. Utilisation de mélanges d'esters de formules I et II selon la revendication 1 pour stabiliser des matières plastiques contre les dégradations photochimiques.

8. Utilisation selon la revendication 1 pour stabiliser des peintures.

9. Procédé de stabilisaion de matières plastiques contre les dégradations photochimiques, procédé selon lequel on ajoute à ces matière 0,01 à 5% en poids d'un mélange des esters de formules I et II selon la revendication 1.

10. Matières plastiques stabilisées contre les dégradations photochimiques, qui contiennent comme stabilisant 0,1 à 5% en poids d'un mélange des esters de formules I et II selon la revendication 1.

11. Matières plastiques stabilisées selon la revendication 10 qui se trouvent dans des peintures.

12. Procédé de préparation de mélanges d'esters de formules I et II selon la revendication 1, procédé caractérisé en ce que l'on fait réagir une pipéridine de formule III avec un diester de formule IV

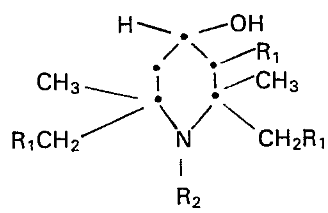

(III)

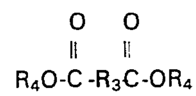

(IV)

les divers symboles ayant les significations données à la revendication 1.